# EUROPEAN PATENT APPLICATION

(11) **EP 4 546 274 A1**
(43) Date of publication of application: **30.04.2025**
(21) Application number: 23205352.0
(22) Date of filing: 23.10.2023
(51) Int. Cl.: G06T 19/00, A61B 17/00, A61B 34/00

(54) **LAPAROSCOPIC IMAGE MANIPULATION METHOD AND SYSTEM AND COMPUTER PROGRAM**

(71) Applicant: OLYMPUS WINTER & IBE GMBH, 22045 Hamburg (DE)
(72) Inventor: SVERDLOV, Juri, 22089 Hamburg (DE); HÜBER, Mathias, 22299 Hamburg (DE); ENDERS, Borg, 21149 Hamburg (DE); JASKOLA, Sascha, 22767 Hamburg (DE); SUPPA, Per, 22083 Hamburg (DE); KUAN, Llin Ie Clarinda, 22393 Hamburg (DE); NIEBUHR, Jan, 24105 Kiel (DE)
(74) Representative: Seemann & Partner Patentanwälte mbB

(57) **Abstract**

The present disclosure relates to a laparoscopic image manipulation method and system (10) and to a computer program implementing said laparoscopic image manipulation method.

The method comprises capturing a video stream of laparoscopic images (24) of a patient (2) using a laparoscope (12) inserted into the patient (2) during a laparoscopic procedure, feeding the captured laparoscopic images (24) to a video processor (14) configured to adding additional information (28) as overlay over the captured laparoscopic images (24), producing a composite image (20) by rendering a representation (26) of a 3D model (18) of a target organ or structure (30) using a renderer (16) and merging the rendered representation of the 3D model (18) with the captured laparoscopic image (24) and displaying the composite image (20) on a monitor.

## Description

The present disclosure relates to a laparoscopic image manipulation method and system and to a computer program implementing said laparoscopic image manipulation method.

During laparoscopic surgery the video camera is inserted via a trocar into the patient's body and the abdominal cavity is visualized on a 2D, 3D or approximated 3D laparoscopic video monitor. Although this minimally invasive approach is generally beneficial for the patient in terms of less trauma, less blood loss and shorter hospital stay compared to the conventional open surgery approach, it comes at the expense of the loss of depth perception for the surgeon and the missing opportunity for tissue palpation.

Depth perception is important for understanding the spatial relationship of anatomical structures, e.g., the distance of a tumor from a main blood supplying vessel in space, and to decide whether the patient can be operated with the laparoscopic approach or whether in fact an open approach is required. To compensate for the lack of depth perception, oftentimes 3D models reconstructed from a Computer Tomography (CT) scan of the patient are used within the operating room and displayed on a second monitor beside the laparoscopic main monitor. Another approach is to use a 3D printed patient-specific model of the target organ or structure within the operating room. Target structures may, e.g., be blood vessels or blood vessel structures, muscular structures, tendon structures, cartilaginous structures, fascial structures or the like.

However, when accessing the additional information, the surgeon is forced to lose eye contact with the primary surgical situation as shown on the laparoscopic video stream when the additional information is displayed on an additional screen or in a 3D printed model. To avoid a dangerous situation, the surgeon will usually stop the procedure with a time-out. However, this disrupts the procedure and leads to an extended procedure time. The latter, in turn, can be associated with a deterioration in patient outcome.

It is an object to provide means to prevent the above-mentioned problems and enhance patient outcome in laparoscopic procedures.

Such object can be solved by a laparoscopic image manipulation method, the method comprising Capturing a video stream of laparoscopic images of a patient using a laparoscope inserted into the patient during a laparoscopic procedure, feeding the captured laparoscopic images to a video processor configured to adding additional information as an overlay over the captured laparoscopic images, producing a composite image by rendering a representation of a 3D model of a target organ or structure using a renderer and merging the rendered representation of the 3D model with the captured laparoscopic image, and displaying the composite image on a monitor. Said monitor may be a surgical main monitor.

The present method provides for a rendering of a 3D model that has been prepared prior to the laparoscopic procedure to be displayed along with the captured laparoscopic images on a monitor, in particular the surgical main monitor. The surgical main monitor is the primary monitor on which all the most relevant data and surgical images are displayed to the surgeon, who is tasked with carrying out the procedure on a patient. Having the rendering of the 3D model displayed on the surgical main monitor within the laparoscopic images means that the surgeon does not have to break eye contact with the laparoscopic image when checking with the rendering of the 3D model. This in turn will obviate the necessity to halt the procedure in order to allow the surgeon to check a rendering of the 3D model being displayed on a separate display or as a physical 3D model of the target organ or structure.

The monitor, in particular surgical main monitor, may be the conventional display, such as a computer monitor. It may also be a heads-up display or a visor display to be worn by the surgeon covering his or her eyes.

In an embodiment, at least one of an orientation, a size and a location of the rendering of the 3D model inside the composite image is controlled by means of a manual controller connected to the renderer. The renderer may be implemented as a rendering software running on the video processor or on a separate computer. A manual controller may be a known controller such as a mouse, a trackball, an Xbox^{®} controller, a PlayStation^{®} controller, a Nintendo Switch^{®} controller, a joystick controller or the like. The manual controller may be controlled by the surgeon performing the procedure or by an assistant keeping track of the orientation of the target organ or structure within the laparoscopic images. The manual manipulation of the orientation and/or the size of the rendering of the 3D model allows the surgeon freedom in assessing the target organ or structure from various angles that are not necessarily accessible with the laparoscope.

In embodiments, information of each individual frame of the video stream of laparoscopic images, in particular image resolution and/or frame rates, are input to the renderer, the renderer being configured to render the representation of the 3D model according to the input information of the individual frames. Having the rendered representation, also called the rendering, of the 3D model synchronized with the individual frames of the video stream of the laparoscopic images ensures that the production of the composite images can proceed in real-time and without the need to reprocess the renderings in order to make them fit into the laparoscopic images.

In the case of 3D images of the laparoscopy produced by using a stereo laparoscope, it is envisioned to render the model in 3D and, when forming the composite images, to merge the left and right renderings of the 3D model with a greater disparity than the structures in the left and right laparoscopic images. The choice of a greater disparity or displacement makes the rendering of the 3D model seem to hover over the image of target organ or structure and surrounding tissues.

In embodiments, the 3D model of the target organ or structure is derived from prior CT and/or MRI scan data of the patient. Such prior CT or MRI scans of the patient may have been made in the radiology department of a hospital and processed at the hospital or by an external provider to generate a 3D model of the target organ or structure. The 3D model of the target organ or structure may be uploaded into the renderer that is configured to render twodimensional representations of the 3D model according to the chosen orientation of the 3D model in space. The 3D model might also be modified in terms of visualization, for example, to reduce any deformation it has encountered during the taking of the prior scans.

The object can also be solved by a laparoscopic image manipulation system comprising a laparoscope, a video processor, a controller and a monitor, in particular a surgical main monitor, the laparoscope being configured to capturing a video stream of laparoscopic images of a patient and feeding the captured laparoscopic images to the video processor, one of the controller, the video processor or a separate computer running a rendering software configured to rendering a representation of a 3D model of a target organ or structure, and the video processor being configured to produce a composite image by merging the rendered representation of the 3D model with the captured laparoscopic image, and the monitor being configured to displaying the composite image.

The system embodies the same features, properties and advantages as the afore described method, providing the surgeon performing a laparoscopy with a composite image displaying a 3D model of a target organ or structure prepared from prior CT or MRI scan data inside the laparoscopic images of the target organ or structure and surrounding tissue in order to provide him with a side-by-side view of the model and the target organ or structure. The surgeon does not need to to take his eyes off the actual laparoscopic image, thus avoiding losing eye contact with the image including a possible following loss of orientation in the field of operation and to have to interrupt the laparoscopic procedure, ensuring a more speedy laparoscopy and a better patient outcome.

The system may comprise a manual controller having a data link to at least one of the controller, the video processor and the separate computer running the rendering software, the rendering software being configured to changing at least one of an orientation, a size and a location of the rendering of the 3D model of the target organ or structure inside the composite image in response to signals from the manual controller. Such manual controller may be a known controller such as a mouse, a trackball, an Xbox^{®} controller, a PlayStation^{®} controller, a joystick controller or the like. The manual controller may be controlled by the surgeon performing the procedure or by an assistant keeping track of the orientation of the target organ or structure within the laparoscopic images.

In embodiments, the system may further comprise a frame grabber configured to capture the laparoscopic video stream frame-by-frame and to produce the composite images by merging of the rendered representation of the 3D model with the captured laparoscopic images frame-by-frame.

In further embodiments, system components, in particular the controller, the image processor, a separate computer, are configured to carry out a laparoscopic image manipulation method according to the previous disclosure.

The above-described objects may also be achieved by a computer program stored on a non-volatile medium, the computer program being designed to perform the steps of the above-described method, in particular when run on a system component of a system according to the previous disclosure. Such computer program embodies the software aspects of the present invention. Different parts of the computer software may be stored and running on different components of the system according to their respective function.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: an embodiment of a laparoscopic image manipulation system,
- Fig. 2: an embodiment of a composite image according to an embodiment of the laparoscopic image manipulation method,
- Fig. 3: further embodiments of composite images and
- Fig. 4: embodiments of a laparoscopic image manipulation method.

In the drawings, the same or similar types of elements or respectively corresponding parts are provided with the same reference numbers in order to prevent the item from needing to be reintroduced.

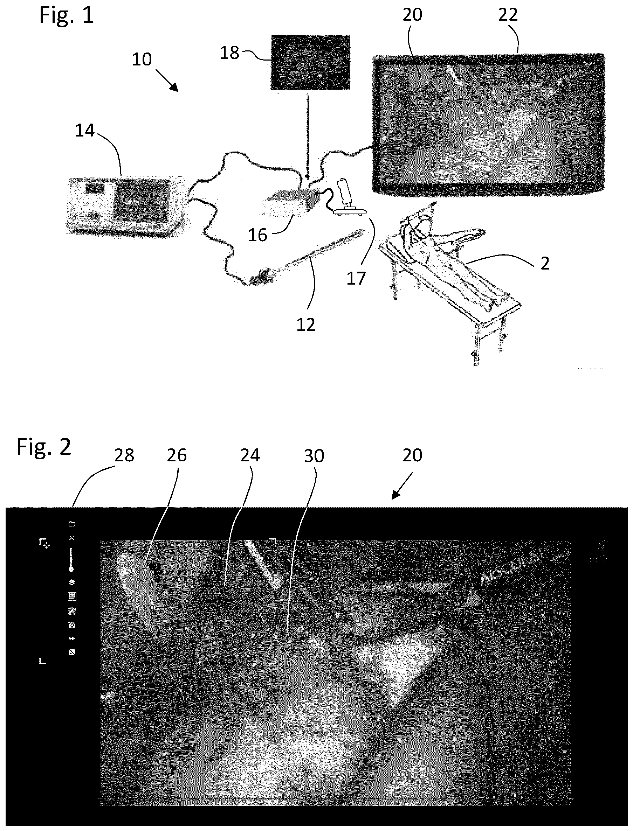

Fig. 1 illustrates an embodiment of a laparoscopic image manipulation system 10. Laparoscopic image manipulation system 10 is set up to be used for a laparoscopy of the patient 2 using a laparoscope 12 which is either equipped with an imaging sensor at the tip of the laparoscope or a camera head (not shown) and its proximal end. Images of a video stream created by the laparoscope 12 are fed into a video processor 14. The output of video processor 14 is fed into a rendering computer 16 configured to use the input of a 3D model 18 to render a representation of the 3D model in a specific orientation and size, which is then merged into the laparoscopic images by overlaying or other means, either within video processor 14 or rendering computer 16 or a central controller (not shown), thereby creating composite image 20, which is displayed on a monitor, which may be a surgical main monitor 22. The system 10 may also comprise a frame grabbing device (not shown) and/or manual controller 17. The manual controller 17 may be used to control the orientation size and or position of the rendering of the 3D model 18 inside composite image. The manual controller 17 may be controlled by the surgeon performing the laparoscopy or by an assistant to the surgeon.

Fig. 2 illustrates an embodiment of a composite image according to an embodiment of the laparoscopic image manipulation method. Composite image 20 is essentially the same as shown in Fig. 1, but with different representation of colors as grayscale hues, in order to better highlight the present disclosure. Composite image 20 comprises the laparoscopic image 24 as provided by laparoscope 12 embedded into a frame which provides additional information and control icons 28. In the upper left corner of laparoscopic image 24, a rendering of a 3D model of the target organ or structure 30 is overlaid over laparoscopic image 24. The target organ or structure 30 as well as the rendering 26 of the 3D model of the target organ or structure are provided with an auxiliary line that the assistant to the surgeon or the surgeon can use to manipulate the orientation of the rendering to conform to the orientation of the real target organ or structure 30 within laparoscopic image 24.

As can be seen in Fig. 2, the rendering 26 of the 3D model of the target organ or structure is clearly distinct from laparoscopic image 24. It is rendered at a different scale than the target organ or structure 30 in laparoscopic image 24 and seemingly hovering above the organs visible in laparoscopic image 24. However, the scale may be changed to match the target organ or structure in the video image or even bigger using manual controller 17. Its surface texture may also intentionally be made to have a more artificial appearance in order to make it immediately recognizable as the model rendering 26 of the target organ or structure 30. Its purpose is to give the surgeon a very clear and quick means of orientation with respect to target organ or structure 30 in order to provide him with the information gained from prior CT or MRI scans of target organ or structure 30 and any lesions, tumors or other abnormalities to be dealt with during the laparoscopy.

In the case of 3D images of the laparoscopy produced by using a stereo laparoscope, it is envisioned to render the model in 3D and, when forming the composite images, to actually make the rendering of the 3D model seem to hover over the image of target organ or structure 30 and surrounding tissues.

Using manual controller 17, the surgeon or assistant can adjust the orientation, the size and/or position of the rendering 26 of the 3D model within the composite image. The choice of orientation of the rendering 26 of the 3D model may be informed by the desire to look at the 3D model from a perspective that is not available with the laparoscope in the laparoscopic image 24, or by the desire to have the orientation of the rendering 26 of the 3D model match the orientation of the target organ or structure 30 in laparoscopic image 24. The choice of size of the rendering 26 of the 3D model may be informed by the desire to look at details by enlarging the rendering 26 of the 3D model and reducing the magnification of the rendering 26 after the inspection of the details has been completed, so that the rendering 26 of the 3D model provides minimal obstruction of laparoscopic image 24. The location of the rendering 26 of the 3D model inside the laparoscopic image 24 may be chosen such as to minimize obstruction of the laparoscopic image 24, in particular of the target organ or structure 30 therein. However, the rendering may also be made semitransparent and overlaid directly over the original target organ or structure 30.

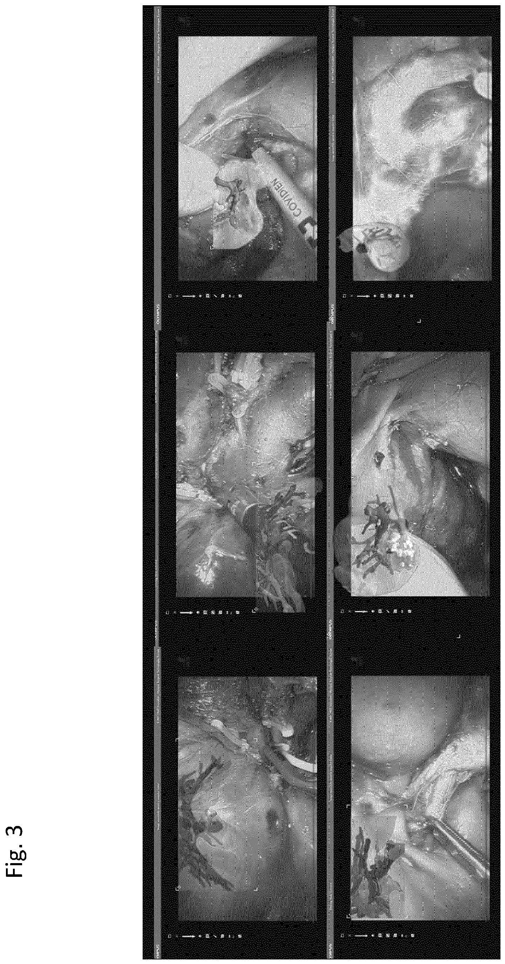

Fig. 3 illustrates further embodiments of composite images, in which renderings of 3D models are superimposed over laparoscopic images. As was the case in Fig. 2, the 3D model of the target organ or structure is displayed directly on the surgical main monitor 22 and can be used in a variety of ways to support intraoperative orientation of the surgeon. For example, it can be used to visualize subsurface structures that cannot be seen in the laparoscopic video image. Such subsurface structures are shown in the screenshots Fig. 3.

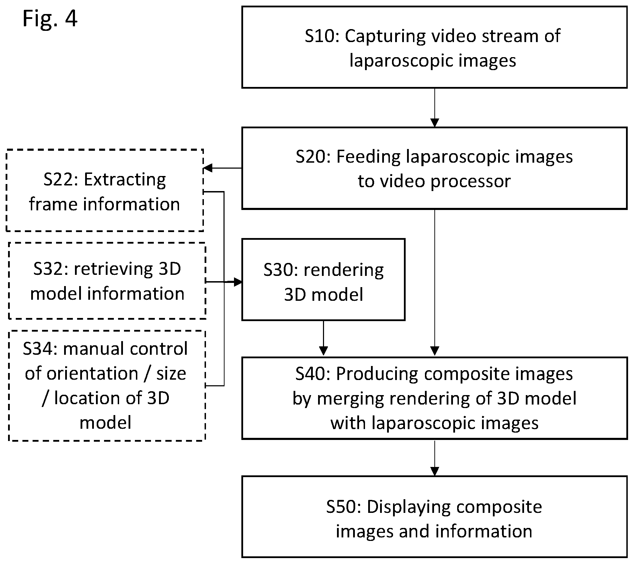

Fig. 4 illustrates embodiments of a laparoscopic image manipulation method. The basic method comprises the steps of capturing a video stream of laparoscopic images (step S10) and feeding the laparoscopic images to a video processor (step S20). In parallel, renderings of a 3D model of the target organ or structure, which may include subsurface structures such as blood vessels, are produced (step S30) and composite images are produced by merging the rendering of the 3D model with the previously captured laparoscopic images (step S40). The composite images and additional information and/or control icons are then displayed on the monitor, in particular the surgical main monitor (step S50).

The rendering of the 3D model in step S30 may have several inputs. This may include retrieving 3D model information (step S32) that have been prepared using previous CT or MRI scans of the target organ or structure and processed to be useful for rendering. The 3D model information may be input once at the start of the laparoscopy. An enhanced synchronicity between the renderings of the 3D model and the laparoscopic images into which the renderings are merged may be achieved by extracting frame information (step S22) from the laparoscopic video stream on a frame-by-frame basis. Furthermore the orientation, size and/or location of the rendering of the 3D model inside the laparoscopic image may be controlled manually (step S34) using a manual controller 17 as described hereinabove.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 2: patient
- 10: laparoscopic image manipulation system
- 12: laparoscope
- 14: video processor
- 16: rendering computer
- 17: manual controller
- 18: 3D model
- 20: composite image
- 22: surgical main monitor
- 24: laparoscopic image
- 26: rendering of 3D model
- 28: additional information
- 30: target organ or structure

## Claims

1. Laparoscopic image manipulation method, the method comprising:
Capturing a video stream of laparoscopic images (24) of a patient (2) using a laparoscope (12) inserted into the patient (2) during a laparoscopic procedure,
Feeding the captured laparoscopic images (24) to a video processor (14) configured to adding additional information (28) as overlay over the captured laparoscopic images (24),
Producing a composite image (20) by rendering a representation (26) of a 3D model (18) of a target organ or structure (30) using a renderer and merging the rendered representation of the 3D model (18) with the captured laparoscopic image (24), and
Displaying the composite image (20) on a monitor, in particular a surgical main monitor (22).

2. The laparoscopic image manipulation method of claim 1, wherein at least one of an orientation, a size and a location of the rendering (26) of the 3D model (18) inside the composite image (20) is controlled by means of a manual controller (17) connected to the renderer (16).

3. The laparoscopic image manipulation method of claim 1 or 2, wherein information of each individual frame of the video stream of laparoscopic images (24), in particular image resolution and/or frame rates, are input to the renderer (16), the renderer (16) being configured to render the representation (26) of the 3D model (18) according to the input information of the individual frames.

4. The laparoscopic image manipulation method of one of claims 1 to 3, wherein the 3D model (18) of the target organ or structure (30) is derived from prior CT and/or MRI scan data of the patient (2).

5. Laparoscopic image manipulation system (10) comprising a laparoscope (12), a video processor (14), a controller and a monitor, in particular a surgical main monitor (22), the laparoscope (12) being configured to capturing a video stream of laparoscopic images (24) of a patient (2) and feeding the captured laparoscopic images (24) to the video processor (14), one of the controller, the video processor (14) or a separate computer running a rendering software configured to rendering a representation (269 of a 3D model (18) of a target organ or structure (30), and the video processor (14) being configured to produce a composite image (20) by merging the rendered representation (26) of the 3D model (18) with the captured laparoscopic image (24), and the monitor being configured to displaying the composite image (20).

6. The laparoscopic image manipulation system (10) of claim 5, further comprising a manual controller (17) having a data link to at least one of the controller, the video processor (14) and the separate computer running the rendering software, the rendering software being configured to changing at least one of an orientation, a size and a location of the rendering (26) of the 3D model (18) of the target organ or structure (30) inside the composite image (20) in response to signals from the manual controller (17).

7. The laparoscopic image manipulation system (10) of claim 5 or 6, further comprising a frame grabber configured to capture the laparoscopic video stream frame-by-frame and to produce the composite images (20) by merging of the rendered representation (26) of the 3D model (18) with the captured laparoscopic images (24) frame-by-frame.

8. The laparoscopic image manipulation system (10) of one of claims 5 to 7, wherein system components, in particular the controller, the image processor, a separate computer, are configured to carry out a laparoscopic image manipulation method according to one of claims 1 to 4.

9. Computer program stored on a non-volatile medium, the computer program being designed to perform the steps of any of claims 1 to 4, in particular when run on a system component of a system (10) according to any of claims 5 to 8.
